# EUROPEAN PATENT SPECIFICATION

(11) **EP 4 652 939 B1**
(45) Date of publication and mention of the grant of the patent: **02.12.2026**
(21) Application number: 25208551.9
(22) Date of filing: 05.04.2024
(51) Int. Cl.: A61B 17/17, A61B 17/00, A61B 34/20, A61B 90/00

(54) **SURGICAL DRILL GUIDE**
CHIRURGISCHE BOHRFÜHRUNG
GUIDE DE FORET CHIRURGICAL

(30) Priority: 05.04.2023 WO PCT/EP2023/059030
(43) Date of publication of application: 26.11.2025
(62) Document number(s) of the earlier application(s) in accordance with Art. 76 EPC: 24715640.9 / 4 568 593
(73) Proprietor: Brainlab SE, 81829 München (DE)
(72) Inventor: Horper, Michael, Munich (DE); Marpert, Eric, Munich (DE); Ayala, Andrew, Munich (DE); Heigl, Rupert, Munich (DE)
(74) Representative: SSM Sandmair

(56) References cited:
- EP-A1- 1 374 784
- EP-B1- 2 227 152
- US-A1- 2009 287 219
- US-A1- 2014 155 905
- US-A1- 2017 333 056
- US-A1- 2019 183 516

## Description

### FIELD OF THE INVENTION

The present invention relates to a device for guiding a surgical drill along a desired trajectory during surgical interventions.

### TECHNICAL BACKGROUND

Drill guide instruments are commonly used in orthopaedics to ensure an accurate and precise operation of surgical drills and to prevent the drill from drifting or deviating from an intended trajectory so as to ensure that the desired location, orientation, size and shape of drill holes is accomplished.

US2009/287219 A1 and EP2227152 B1 disclose surgical drill guides.

Known drill guide instruments are however cumbersome and difficult for users to handle.

The present invention has the object of providing a surgical drill guide that comprises a trocar insert and which is easy to handle and operate.

### SHORT DESCRIPTION OF THE INVENTION

The surgical drill guide according to the invention is defined in claim 1. Further embodiments of the invention are recited in the dependent claims.

In the following, a short description of the specific features of the present disclosure is given which shall not be understood to limit the invention only to the features or a combination of the features described in this section.

In general, the present disclosure relates to a surgical drill guide comprising
- an instrument body member having a first guide channel configured to receive a surgical drill bit; and
- a depth control member having a proximal drill stop surface and a second guide channel configured to receive the surgical drill bit, wherein the depth control member and the instrument body member connect to a rotatable intermediate member via respective interface sections, such that the second guide channel is disposed proximal to the first guide channel; and
- a trocar insert configured for being received in the first and the second guide channel, which releasably connects to a proximal section of the intermediate member. Various aspects which improve handling and operation of the inventive drill guide are disclosed in the following description part.

### GENERAL DESCRIPTION OF THE DISCLOSURE

In this section, a description of the general features of the present disclosure is given for example by referring to possible aspects and embodiments of the disclosure.

In general, the disclosure reaches the aforementioned object by providing a surgical drill guide with an instrument body member having a first guide channel configured to receive a surgical drill bit, wherein the drill guide is characterized by advantageous features in several aspects. Each one of these aspects can be configured as standalone which is independent from the remaining aspects. However. different aspects of the present examples and their embodiments which are discloses in the following can be combined wherever technically expedient and feasible.

In a further embodiment, the first guide channel is provided by an instrument tip member which releasably connects to the instrument body member, particularly wherein a spring-loaded mechanism holds the instrument tip member in place with respect to the instrument body member via a form-fit connection.

### Trocar Insert

A first aspect of the present disclosure relates to a surgical drill guide comprising
- an instrument body member having a first guide channel configured to receive a surgical drill bit;
- a depth control member having a proximal drill stop surface and a second guide channel configured to receive the surgical drill bit, wherein the depth control member and the instrument body member are coupled to each other via the rotatable intermediate member, such that the second guide channel is disposed proximal to the first guide channel, and wherein the surgical drill guide further comprises
- a trocar insert configured for being received in the first and the second guide channel, which releasably connects to a proximal section of the intermediate member, particularly via a snap-fit connection, which is specifically adapted to release upon a separation force in the proximal-distal-direction, which is less than a separation force which the snap-fit connection of the rotary joint is adapted to release upon.

As soon as the tip portion of the trocar is pushed against a bony structure, the force applied onto the trocar may cause the snap-fit connection between the intermediate member and the trocar to disengage, with the snap-fit connection between the intermediate member and the instrument body member persisting.

Such trocar insert helps in advancing the surgical drill guide, i.e. its distal shaft section through tissue. For example, the trocar insert may have a tapered tip at its distal end which offers less resistance when being advanced through tissue than the tubular tip of the drill guide. In case the distal tip of the drill guide features one or more cutting edges, the distal portion of the trocar insert may be configured to cover one side of the cutting edges, thereby eliminating its cutting properties and the risk of unwanted harm to tissue while the drill guide is advanced through tissue.

For example, the trocar insert may have a proximal portion which may not only be configured to releasably engage with a proximal portion of the intermediate member. It may further be configured to engage an open channel or groove of the depth control member along a proximal-distal-direction. In general, the trocar insert and the depth control member may be configured to allow for the trocar insert to be advanced through the first and the second guide channel and to be subsequently fixed via its proximal portion, to a proximal portion of the intermediate member, irrespective of the relative position between the depth control member and the instrument body member along the proximal-distal direction.

### Depth Stop

A further aspect of the present disclosure relates to a surgical drill guide comprising, in addition to the instrument body member having a first guide channel configured to receive a surgical drill bit, a depth control member having a proximal drill stop surface and a second guide channel configured to receive the surgical drill bit, a depth control member which connects to the instrument body member via a rotatable intermediate member, such that the second guide channel is disposed proximal to the first guide channel, wherein the interface section of the depth control member is configured to engage the rotatable intermediate member via a threaded joint, and wherein the interface section of the instrument body member is configured to engage the rotatable intermediate member via a rotary joint allowing the rotatable intermediate member to rotate with respect to the instrument body member and around a rotational axis extending in a proximal-distal-direction of the drill guide, such that the depth control member moves relative to the instrument body member along the proximal-distal-direction as the rotatable intermediate member rotates with respect to the instrument body member.

In other words, the rotatable intermediate member interconnects the instrument body member and the depth control member such that the depth control member is held firmly in place along the proximal-distal-direction and with respect to the instrument body member as long as the intermediate member is not rotated. As soon as the intermediate member is rotated around its rotational axis, though, the threaded joint between the intermediate member and the depth control member causes the depth control member to move along the proximal-distal-direction. Depending on the direction in which the intermediate member is rotated, the depth control member either moves in a distal or in a proximal direction. It needs to be noted here that the friction occurring between the respective parts of the rotary joint and/or of the threaded joint is large enough to prevent an unintended rotation of the intermediate member around its rotational axis. Rather, the intermediate member needs to be grasped by a person and turned round its rotational axis to move the depth control member with respect to the instrument body member. Either one or both of the rotary joint and the threaded joint may be configured as self-impeding joint in which friction increases rapidly as soon as force is transmitted between the depth control member and the instrument body member along the proximal-distal-direction. With the above-described mechanism, the relative position between the depth control member and the instrument body member along the proximal-distal-direction can be easily adjusted by simply rotating the intermediate member, wherein a set relative position is safely maintained by the intermediate member.

In another embodiment, the rotary joint includes a releasable connection, particularly a snap-fit connection between the rotatable intermediate member and the instrument body member. Such releasable connection allows for quickly releasing the intermediate member along with the depth control member from the instrument body member without the need of rotating the intermediate member until the depth control member has moved all the way in the proximal direction until it leaves the threaded engagement with the intermediate member. With this specific embodiment, it is possible to quickly swap the depth control member with another depth control member which may have a different configuration, for example a different length.

In another embodiment, the depth control member comprises
at least one first surface segment extending along the length of the depth control member and providing the threaded joint; and/or
at least one second surface segment extending along the length of the depth control member and providing a surface adapted to contact a corresponding surface of the instrument body member to prevent the depth control member from rotating with respect to the instrument body member and/or to form a sliding guide for the depth control member as it moves along the proximal-distal-direction. With this embodiment, the depth control member and the intermediate member mesh with each other only in predefined sections of the depth control member's outer circumference, whilst the remaining parts of the depth control members outer circumference serve as anti-twist protection and/or slide-guide for the pairing between the depth control member and the instrument body member.

In a further embodiment, the rotatable intermediate member has the shape of a substantially cylindrical sleeve having an inner thread;
- the outer circumferential surface of the rotatable intermediate member is configured to provide a gripping surface;
- the rotatable intermediate member includes at least one inspection window in which the depth control member, particularly a length scale thereof is visible; and/or
- the proximal end portion of the rotatable intermediate member features a larger inner diameter than the remaining rotatable intermediate member, particularly the inner thread thereof, allowing the proximal portion of the depth control member to enter the proximal portion of the rotatable intermediate member.

In a further embodiment, the depth control member is partially received within the instrument body member, particularly wherein the depth control member is adapted to be telescoped out of and into the instrument body member as the rotatable intermediate member is rotated.

In a further embodiment, the proximal section of the depth control member features an open cross section. As will be described further below in more detail, a depth control member having an open cross section, i.e. an open guide channel at its proximal section allows for a trocar to be entered into the guide channels and subsequently locked in position at a proximal portion of the intermediate member, regardless of the position along the proximal-distal-direction at which the depth control member is fixed to the instrument body member.

### Tracking Reference Member

A further aspect of the present disclosure relates to a surgical drill guide comprising
- an instrument body member having a first guide channel configured to receive a surgical drill bit;
- a depth control member having a proximal drill stop surface and a second guide channel configured to receive the surgical drill bit, wherein the depth control member and the instrument body member connect to each other via the rotatable intermediate member, such that the second guide channel is disposed proximal to the first guide channel, and wherein the surgical drill guide further comprises
- a tracking reference member, wherein the tracking reference member and the instrument body member releasably connect to each other via corresponding reference interface sections joining together in at least one form-fit connection as well as in at least one force-fit connection.

In other words, the tracking reference member is not only held in place with respect to the instrument body member via at least one form-fit connection, but additionally via a force-fit connection.

In one embodiment, the at least one form-fit connection defines a location and/or orientation, particularly exactly one location and exactly one orientation in which the tracking reference member and the instrument body member connect to each other and/or is provided by correspondingly shaped surfaces at which the reference interface section contact each other.

This allows for the use of pre-calibrated tracking references as the relative position between the tracking reference member and the instrument body member is precisely defined by the form-fit connection established between the tracking reference member and the instrument body member.

In a further embodiment, the at least one force-fit connection includes
- a screw connection, particularly with an inner thread provided by the instrument body member, holding the tracking reference member in place with respect to the instrument body member and/or
- magnetic elements disposed at the respective reference interface sections, holding the tracking reference member in place with respect to the instrument body member.

While the screw connection establishes a rigid but still releasable connection between the tracking reference member and the instrument body member, the force-fit connection provided by said magnetic elements may aid in attaching the tracking reference member to the instrument body member. Even with a loose screw connection, the tracking reference member is held in place via the magnetic elements, thereby preventing the tracking reference member from being unintentionally disconnected from the instrument body member which may cause the tracking reference member being dropped and damaged.

### Handle Member

A further aspect of the present disclosure relates to a surgical drill guide comprising
- an instrument body member having a first guide channel configured to receive a surgical drill bit; and
- a depth control member having a proximal drill stop surface and a second guide channel configured to receive the surgical drill bit, wherein the depth control member and the instrument body member connect to each other via the rotatable intermediate member such that the second guide channel is disposed proximal to the first guide channel, and wherein the surgical drill guide further comprises
- a handle member, wherein the handle member and the instrument body member releasably connect to each other via corresponding handle interface sections which are configured to allow for a plurality of the relative positions, particularly around a rotational axis defined by the handle interface section, in which the handle member connects to the instrument body member.

In other words, the handle member and the instrument body member are configured to allow for adjusting the relative position in which the handle member rigidly connects to the instrument body member.

In a further embodiment, the handle interface sections
- are disposed around the depth control member and the second guide channel defined therein, particularly wherein the proximal section of the instrument body member is disposed radially between the depth control member and the handle interface sections;
- comprise correspondingly shaped surfaces at which the handle interface sections contact each other, wherein the correspondingly shaped surfaces are disposed within a recess formed either in the instrument body member or the handle member; and/or
- join together in a form-fit connection allowing for a predefined number of relative positions.

In a still further embodiment, the handle interface sections are held together by a mechanism including a knob engaging the instrument body member via a bayonet coupling, and a spring member connecting between the knob and the handle member, wherein the mechanism is configured to preload the spring member as the knob engages the instrument body member, thereby forcing the handle interface sections together, particularly wherein the knob provides a shield at a radial position of the open cross section of the second guide channel.

Again, it is to be noted here that each one of the aspects described above can be considered as a separate disclosure which is independent from other aspects and the features thereof. However, the features of any aspect can be combined with features of one or more further aspects wherever technically expedient and feasible.

A further aspect of the present disclosure relates to a drill guide system comprising
- a surgical drill guide as described above;
- two or more depth control members as described above and adapted to connect to the instrument body member and/or two or more instrument tip members as described above and adapted to connect to the instrument body member;
- two or more surgical drill bits,
wherein the first guide channels respectively provided by the at least two depth control members and/or the second guide channels respectively provided by the at least two instrument tip members feature inner diameters which correspond to the outer diameter of a proximal shaft section and/or to the outer diameter of a distal cutting section of the at least one drill bit, such that only specific types of surgical drill bits, which in particular have a specific length, are capable of being received in the first guide channel and/or the second guide channel.

### DEFINITIONS

In this section, definitions for specific terminology used in this disclosure are offered which also form part of the present disclosure.

### Marker

It is the function of a marker to be detected by a marker detection device (for example, a camera or an ultrasound receiver or analytical devices such as CT or MRI devices) in such a way that its spatial position (i.e. its spatial location and/or alignment) can be ascertained. The detection device is for example part of a navigation system. The markers can be active markers. An active marker can for example emit electromagnetic radiation and/or waves which can be in the infrared, visible and/or ultraviolet spectral range. A marker can also however be passive, i.e. can for example reflect electromagnetic radiation in the infrared, visible and/or ultraviolet spectral range or can block x-ray radiation. To this end, the marker can be provided with a surface which has corresponding reflective properties or can be made of metal in order to block the x-ray radiation. It is also possible for a marker to reflect and/or emit electromagnetic radiation and/or waves in the radio frequency range or at ultrasound wavelengths. A marker preferably has a spherical and/or spheroid shape and can therefore be referred to as a marker sphere; markers can however also exhibit a cornered, for example cubic, shape.

### Marker device

A marker device can for example be a reference star or a pointer or a single marker or a plurality of (individual) markers which are then preferably in a predetermined spatial relationship. A marker device comprises one, two, three or more markers, wherein two or more such markers are in a predetermined spatial relationship. This predetermined spatial relationship is for example known to a navigation system and is for example stored in a computer of the navigation system.

In another embodiment, a marker device comprises an optical pattern, for example on a two-dimensional surface. The optical pattern might comprise a plurality of geometric shapes like circles, rectangles and/or triangles. The optical pattern can be identified in an image captured by a camera, and the position of the marker device relative to the camera can be determined from the size of the pattern in the image, the orientation of the pattern in the image and the distortion of the pattern in the image. This allows determining the relative position in up to three rotational dimensions and up to three translational dimensions from a single two-dimensional image.

The position of a marker device can be ascertained, for example by a medical navigation system. If the marker device is attached to an object, such as a bone or a medical instrument, the position of the object can be determined from the position of the marker device and the relative position between the marker device and the object.

Determining this relative position is also referred to as registering the marker device and the object. The marker device or the object can be tracked, which means that the position of the marker device or the object is ascertained twice or more over time.

### Marker holder

A marker holder is understood to mean an attaching device for an individual marker which serves to attach the marker to an instrument, a part of the body and/or a holding element of a reference star, wherein it can be attached such that it is stationary and advantageously such that it can be detached. A marker holder can for example be rod-shaped and/or cylindrical. A fastening device (such as for instance a latching mechanism) for the marker device can be provided at the end of the marker holder facing the marker and assists in placing the marker device on the marker holder in a force fit and/or positive fit.

### Reference star

A "reference star" refers to a device with a number of markers, advantageously three markers, attached to it, wherein the markers are (for example detachably) attached to the reference star such that they are stationary, thus providing a known (and advantageously fixed) position of the markers relative to each other. The position of the markers relative to each other can be individually different for each reference star used within the framework of a surgical navigation method, in order to enable a surgical navigation system to identify the corresponding reference star on the basis of the position of its markers relative to each other. It is therefore also then possible for the objects (for example, instruments and/or parts of a body) to which the reference star is attached to be identified and/or differentiated accordingly. In a surgical navigation method, the reference star serves to attach a plurality of markers to an object (for example, a bone or a medical instrument) in order to be able to detect the position of the object (i.e. its spatial location and/or alignment). Such a reference star for example features a way of being attached to the object (for example, a clamp and/or a thread) and/or a holding element which ensures a distance between the markers and the object (for example in order to assist the visibility of the markers to a marker detection device) and/or marker holders which are mechanically connected to the holding element and which the markers can be attached to.

### Surgical navigation system

A navigation system, such as a surgical navigation system, is understood to mean a system which can comprise: at least one marker device; a transmitter which emits electromagnetic waves and/or radiation and/or ultrasound waves; a receiver which receives electromagnetic waves and/or radiation and/or ultrasound waves; and an electronic data processing device which is connected to the receiver and/or the transmitter, wherein the data processing device (for example, a computer) for example comprises a processor (CPU) and a working memory and advantageously an indicating device for issuing an indication signal (for example, a visual indicating device such as a monitor and/or an audio indicating device such as a loudspeaker and/or a tactile indicating device such as a vibrator) and a permanent data memory, wherein the data processing device processes navigation data forwarded to it by the receiver and can advantageously output guidance information to a user via the indicating device. The navigation data can be stored in the permanent data memory and for example compared with data stored in said memory beforehand.

### BRIEF DESCRIPTION OF THE DRAWINGS

In the following, the invention is described with reference to the appended figures which give background explanations and represent specific embodiments of the invention. The scope of the invention is however not limited to the specific features disclosed in the context of the figures, wherein
- Figure 1: shows a surgical drill guide instrument that forms part of the present invention;
- Figure 2: shows a cross-sectional side view of the drill guide of Figure 1;

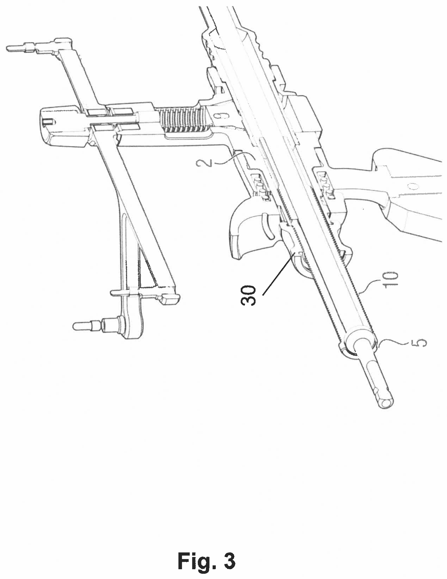

- Figure 3: shows a further cross-sectional view of the drill guide of Figure 1;

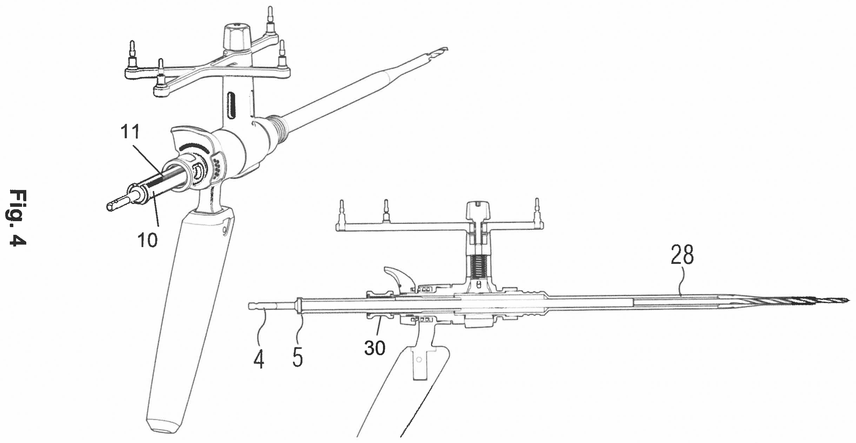

- Figure 4: shows further views of the drill guide of Figure 1;
- Figure 5: shows a trocar insert that forms part of the present disclosure;
- Figure 6: shows a tracking reference member of the drill guide of Figure 1;
- Figure 7: shows a handle member of the drill guide of Figure 1;
- Figure 8: shows various possible positions of the handle member with respect to the instrument body member;
- Figure 9: shows a detailed perspective view on the handle interface sections of Figure 7;
- Figure 10: shows an instrument tip member of the drill guide of Figure 1.

### DESCRIPTION OF EMBODIMENTS

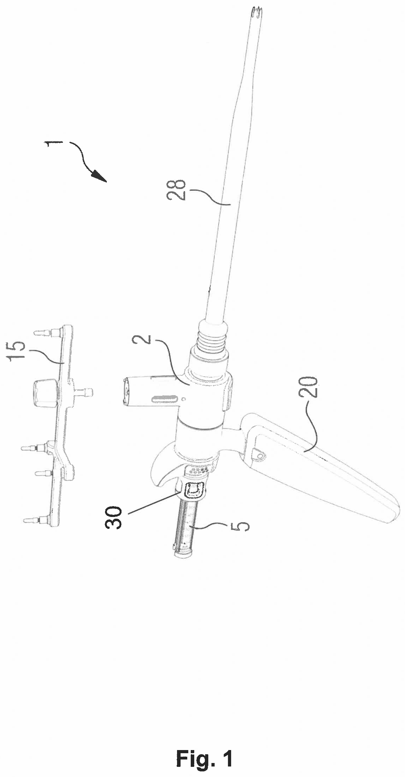

Figure 1 shows a surgical drill guide instrument 1 according to the present invention, which comprises an instrument body member 2, a depth control member 5, a tracking reference member 15, a handle member 20 and an instrument tip member 28, all of which are releasably connected to each other.

The main purpose of the drill guide instrument 1 is to define a trajectory for a drill bit 4 during a surgical drilling operation. While the drill guide 1 is grasped by a user at the handle section 20, the spatial position, i.e. the spatial location and/or the spatial orientation of the drill guide 1 is determined and tracked over time by a medical tracking system configured to recognize a plurality of spherical tracking markers (not shown in the Figures) disposed at corresponding attachment points of the tracking reference member 15.

Further, the drill guide 1 is configured to provide a defined length of a guiding channel for the surgical drill bit 4, which is defined by the distance between the drill stop surface 6 in an axial recess in the proximal section 12 of the depth control member 5 and the distal tip of the instrument tip member 28. This distance is reduced by telescoping the depth control member 5 into the instrument body member 2 in a distal direction and is increased by telescoping the depth control member 5 out of the instrument body member 2 in a proximal direction. As soon as desired relative position between the depth control member 5 and the instrument body member 2 is reached, this relative position is maintained by the rotatable intermediate member 30 in a manner that will be described in more detail further below.

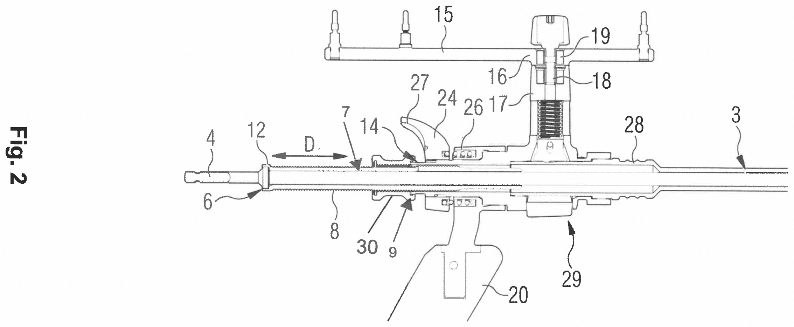

Figure 2 shows a cross sectional side view of the drill guide 1, with a surgical drill bit 4 being introduced into guide channels 7 and 3. As can be also seen, the surgical drill bit 4 can be advanced in a distal direction until a collar section thereof (not indicated) abuts the proximal drill stop surface 6 of the depth control member 5. The length of the drill bit 4 beyond its collar section which exceeds the length defined between the proximal drill stop surface 6 and the distal tip protrudes from the instrument tip member 28 and defines the depth of a hole to be drilled.

The depth control member 5 is received within instrument body member 2 in a rotatably fixed manner and can be moved therein along its longitudinal axis.

As becomes apparent from Figure 2, the rotatable intermediate member 30 connects to the proximal section 14 of the instrument body member 2 in a rotary joint which leaves the intermediate member 30 with only one rotational degree of freedom around the proximal-distal-direction D. On the other hand, the intermediate member 30 features an inner thread which meshes with an outer thread 8 provided in predefined portions on the outer circumference of the depth control member 5, which extend in the proximal-distal-direction. Rotation of the intermediate member 30 around its rotational axis therefore causes the depth control member 5 to move in a proximal or in a distal direction, depending in which direction the intermediate member 30 is rotated.

For a quick release of the intermediate member 30 along with the depth control member 5, the intermediate member 30 is snap-fitted to the proximal section 14 and the rotary joint thereof. At its proximal section, the intermediate member 30 features a further snap-fit-connection to a trocar 13, which is adapted to release as soon as a predefined force is applied to the trocar 13 in the proximal direction, for example as soon as the tip of the trocar 13 is pushed against bony structures.

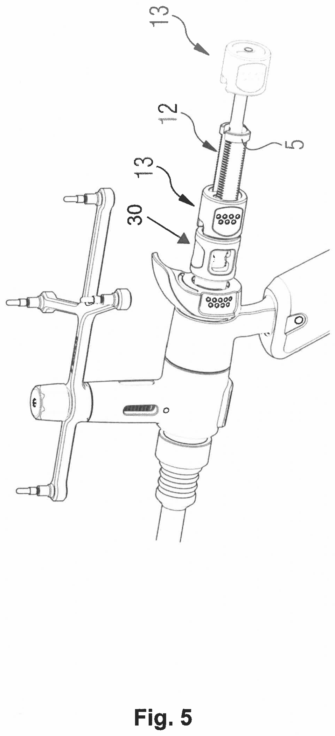

As is also best seen in Figures 5, 10 and 11, the intermediate member 30 features cutouts or windows through which a length-scale of depth control member 5 is seen (cf. Figure 1), such that the length of the drill bit 4 extending from the distal end of the drill guide 1 can be easily determined.

Figure 5 shows a trocar insert 13 configured to be introduced into guide channels 3 and 7 so as to protrude from the distal tip of instrument tip member 28. The trocar insert 13 can be locked into place at the proximal section of the rotatable intermediate member 30 while the drill guide 1 is advanced through tissue towards a desired location within a patient's body.

As becomes apparent from Figure 5, the trocar insert 13 can be introduced and locked in place even with the depth control member 5 being telescoped out of the instrument body member 2. In fact, the installation and de-installation of the trocar insert 13 is entirely independent from the relative position of the depth control member 5 with respect to the instrument body member 2. This is because the guide channel 7 of the depth control member 5 features an open cross section in a proximal section 12, i.e. is formed as a channel or groove which allows for the trocar insert 13 to be advanced in a distal direction past the depth control member 5 until it reaches its locking position at the proximal section of the rotatable intermediate member 30.

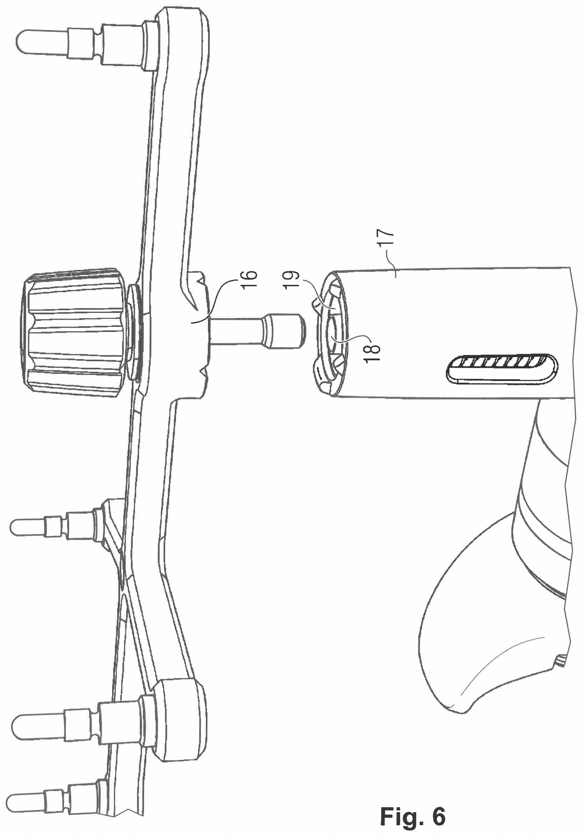

Figure 6 shows a tracking reference member 15 which can be releasably connected to the instrument body member 2 via corresponding interface sections 16 and 17. Interface section 17 comprises several protrusions which extend in an upwards direction and towards the interface section 16 assigned to the tracking reference member 15. In turn, interface section 16 features a plurality of recesses each of which is to receive a correspondingly formed protrusion of interface section 17. This results in one single spatial position in which the tracking reference number 15 can be attached to the instrument body member 2. As can be further seen in Figure 6, interface section 17 comprises a ring-shaped permanent magnet 19 which finds a corresponding counterpart permanent magnet in interface section 16. As soon as both of these permanent magnets are brought into proximity of one another, electromagnetic force pulls interface sections 16 and 17 together, thereby holding tracking reference member 15 attached to instrument body member 2. At this stage, tracking reference member 15 is securely held attached to instrument body member 2 via the permanent magnets until the screw connection between the threaded shaft (not indicated in Figure 6) is screwed into the inner thread 18 provided at interface section 17 which firmly connects tracking reference member 15 and instrument body member 2.

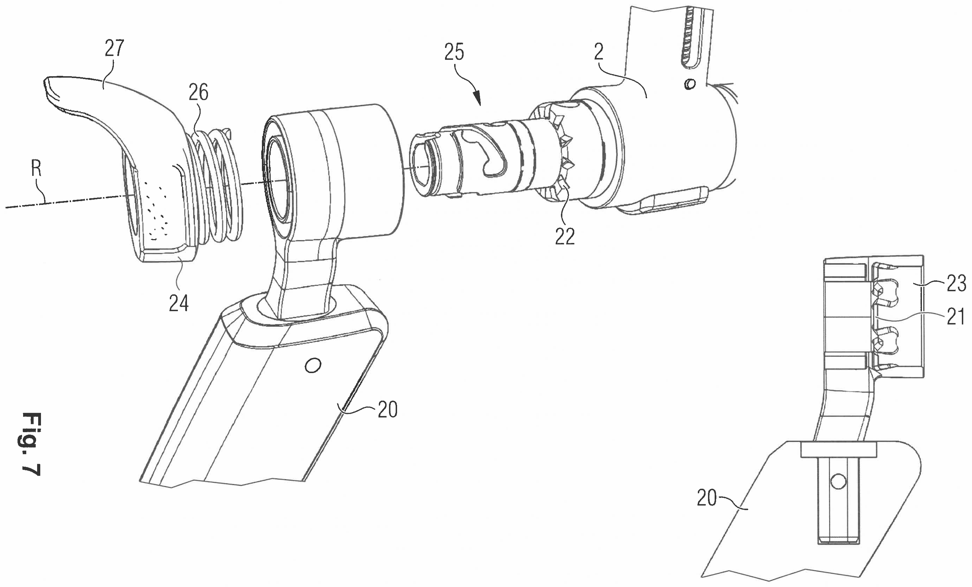

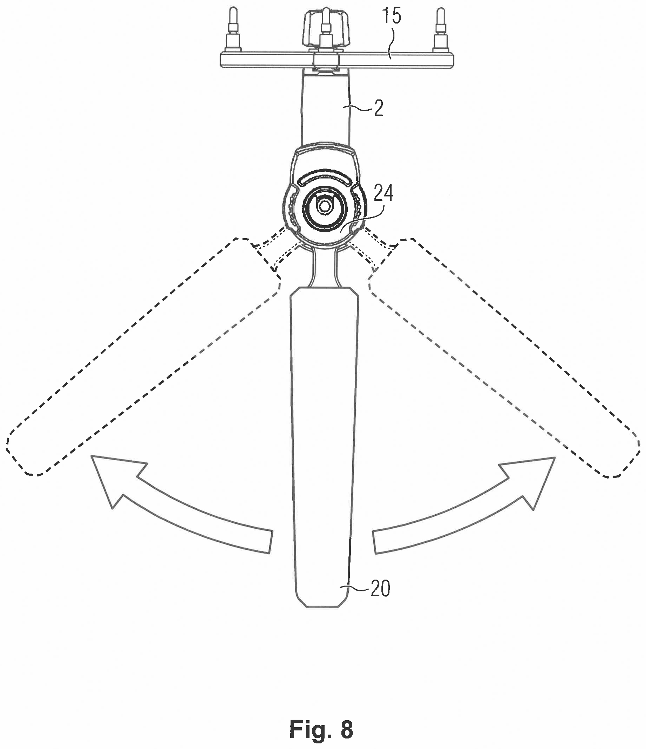

Figure 7 shows handle interface sections 21 and 22 which are to connect handle member 20 to instrument body member 2. Handle section 20 features a ring shaped section through which the proximal section 14 of the instrument body member 2 is forwarded in a proximal direction until the plurality of equally spaced protrusions of interface section 21 is received in correspondingly shaped recesses at handle interface section 22. In order to secure the handle member 20 to the instrument body member 2, knob 24 connects to the proximal section 14 of instrument body member 2 via bayonet-coupling 25. Knob 24 may take two different rotational positions with respect to instrument body member 2. In the first position, coil spring 26 between knob 24 and the ring-shaped section of handle member 20 is forcing the ring-shaped interface sections 21 and 22 firmly together, wherein the correspondingly shaped protrusions and recesses prevent any rotational motion of handle member 20 around axis R and with respect to instrument body member 2. In a second position allowed by the bayonet coupling 25, knob 24 takes a more proximal position with respect to instrument body member 2, in which the spring force caused by coil-spring 26 and acting on handle interface sections 21 and 22 is reduced to such an amount that allows for the protrusions and correspondingly shaped recesses of handle interface sections 21 and 22 to disengage from each other, eventually allowing handle member 20 for being rotated around axis R as shown in Figure 8. Once a desired rotational position of handle member 20 is reached, the user rotates knob 24 back into the first position such that coil spring 26 firmly forces interfaces 21 and 22 together.

Bayonet coupling 25 comprises a longitudinal recess at the outer circumference of proximal section 14 which extends obliquely (cf. Figure 2) between two resting positions, one of which takes a more distal position while the other takes a more proximal position. A protrusion at the inner circumference of knob 24 engages the recess, such that knob 24 is held at the proximal section 14 of instrument body member 2 in both resting positions. Once the protrusion of knob 24 rests in the more proximal resting position, knob 24 can be released from proximal section 14 by turning it even further in an anti-clockwise direction such that the protrusion exits the recess (cf. Figure 7).

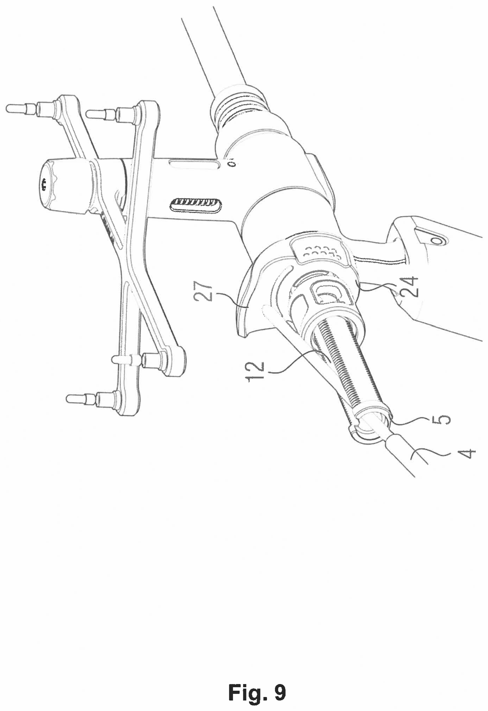

Figure 9 shows a further functionality of knob 24, namely a stabbing protection provided by shield 27 which is integrally formed with knob 24. **In** case drill bit 4 or any other instrument to be introduced into guiding channels 3 and 7 unintentionally leaves the open, groove-shaped proximal section 12 of depth control member 5, shield 27 protects a user's hand grasping instrument body member 2 from being punctured.

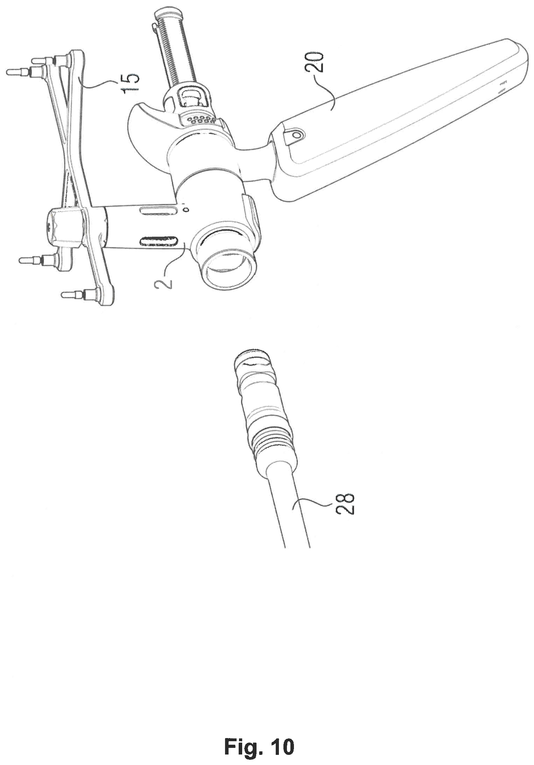

As can be seen from Figure 10, the first guide channel 3 is formed within an instrument tip member which releasably connects to the distal portion of instrument body member 2. Instrument tip member 28 is held in place via a form-fit connection between a circumferential groove at a proximal section of instrument tip member 28 and a protrusion which is held within the circumferential groove by a spring-loaded mechanism 29 (cf. Figure 2). In order to release instrument tip member 28 from instrument body member 2, the spring-loaded mechanism 29 is pushed in an upwards direction such that the protrusion leaves the circumferential groove and instrument tip member 28 can be pulled out of instrument body member 2 in a distal direction. For assembly, instrument tip member 28 is pushed into the distal portion of instrument body member 2, wherein the protrusion automatically snap-fits into the circumferential groove.

## Claims

1. Surgical drill guide comprising
- an instrument body member (2) having a first guide channel (3) configured to receive a surgical drill bit (4); and
- a depth control member (5) having a proximal drill stop surface (6) and a second guide channel (7) configured to receive the surgical drill bit (4);
wherein the depth control member (5) and the instrument body member (2) connect to each other via respective interface sections (8, 9) of a rotatable intermediate member (30), such that the second guide channel (7) is disposed proximal to the first guide channel (3), wherein the surgical drill guide further comprises
- a trocar insert (13) configured for being received in the first and the second guide channel (3, 7), which releasably connects to a proximal section of the intermediate member (30), **characterized in that**
the trocar insert (13) connects to a proximal section of the intermediate member (30) via a snap-fit connection which is adapted to release upon a separation force in the proximal-distal-direction, which is less than a separation force which a snap-fit connection between the intermediate member (30) and the instrument body member (2) is adapted to release upon.

2. The surgical drill guide according to claim 1, wherein the interface section (9) of the instrument body member (2) is configured to engage the rotatable intermediate member (30) via a rotary joint allowing the rotatable intermediate member (30) to rotate with respect to the instrument body member (2) and around a rotational axis extending in a proximal-distal-direction (D) of the drill guide (1), and wherein the rotary joint includes the snap-fit connection between the rotatable intermediate member (30) and the instrument body member (2).

3. The surgical drill guide according to any one of claims 1 and 2, wherein
- the rotatable intermediate member (30) has the shape of a substantially cylindrical sleeve having an inner thread;
- the outer circumferential surface of the rotatable intermediate member (30) is configured to provide a gripping surface;
- the rotatable intermediate member (30) includes at least one inspection window in which the depth control member (5), particularly a length scale thereof, is visible; and/or
- the proximal end portion of the rotatable intermediate member (30) features a larger inner diameter than the remaining rotatable intermediate member (30), particularly the inner thread thereof, allowing the proximal portion of the depth control member (5) to enter the proximal portion of the rotatable intermediate member (30).

4. The surgical drill guide according to any one of claims 1 to 3, further comprising a tracking reference member (15), wherein the tracking reference member (15) and the instrument body member (2) releasably connect to each other via corresponding reference interface sections (16, 17) joining together in at least one form-fit connection as well as in at least one force-fit connection.

5. The surgical drill guide according to claim 4, wherein at least one form-fit connection defines a location and/or orientation, particularly exactly one location and exactly one orientation in which the tracking reference member (15) and the instrument body member (2) connect to each other and/or is provided by correspondingly shaped surfaces at which the reference interface sections (16, 17) contact each other.

6. The surgical drill guide according to any one of claims 4 and 5, wherein the at least one force-fit connection includes
- a screw connection, particularly with an inner thread (18) provided by the instrument body member (2), holding the tracking reference member (15) in place with respect to the instrument body member (2) and/or
- magnetic elements (19) disposed at the respective reference interface sections (16, 17), holding the tracking reference member (15) in place with respect to the instrument body member (2).

7. The surgical drill guide according to any one of claims 1 to 6, further comprising a handle member (20), wherein the handle member (20) and the instrument body member (2) releasably connect to each other via corresponding handle interface sections (21, 22) which are configured to allow for a plurality of relative positions, particularly around a rotational axis (R) defined by the handle interface sections (21, 22), in which the handle member (20) connects to the instrument body member (2).

8. The surgical drill guide according to claim 7, wherein the handle interface sections (21, 22)
- are disposed around the depth control member (5) and the second guide channel (7) defined therein, particularly wherein the proximal section (14) of the instrument body member (2) is disposed radially between the depth control member (5) and the handle interface sections (21, 22);
- comprise correspondingly shaped surfaces at which the handle interface sections (21, 22) contact each other, wherein the correspondingly shaped surfaces are disposed within a recess (23) formed either in the instrument body member (2) or the handle member (20); and/or
- join together in a form-fit connection allowing for a predefined number of relative positions.

9. The surgical drill guide according to any one of claims 7 and 8, wherein the handle interface sections (21, 22) are held together by a mechanism including a knob (24) engaging the instrument body member (2) via a bayonet coupling (25), and a spring member (26) connecting between the knob (24) and the handle member(20), wherein the mechanism is configured to preload the spring member (26) as the knob (24) engages the instrument body member (2), thereby forcing the handle interface sections (21, 22) together, particularly wherein the knob (24) provides a shield (27) at a radial position of the open cross section (12) of the second guide channel (7).

10. The surgical drill guide according to any one of claims 1 to 9, wherein the first guide channel (3) is provided by an instrument tip member (28) which releasably connects to the instrument body member (2), particularly wherein a spring-loaded mechanism (29) holds the instrument tip member (28) in place with respect to the instrument body member (2) via a form-fit connection.

11. Drill guide system comprising
- a surgical drill guide (1) according to any one of claims 1 to 10;
- two or more depth control members (5) adapted to connect to the instrument body member (2) and/or two or more instrument tip members (28) adapted to connect to the instrument body member (2);
- two or more surgical drill bits (4);
wherein the first guide channels (3) respectively provided by the at least two depth control members (5) and/or the second guide channels (7) respectively provided by the at least two instrument tip members (28) feature inner diameters which correspond to the outer diameter of a proximal shaft section and/or to the outer diameter of a distal cutting section of the at least one drill bit (4), such that only specific types of surgical drill bits (4), which in particular have a specific length, are capable of being received in the first guide channel (3) and/or the second guide channel (7).

## Patentansprüche

1. Chirurgische Bohrführung, umfassend
- ein Instrumentenkörperelement (2) mit einem ersten Führungskanal (3), der zur Aufnahme eines chirurgischen Bohrers (4) ausgebildet ist; und
- ein Tiefenkontrollelement (5) mit einer proximalen Bohranschlagfläche (6) und einem zweiten Führungskanal (7), der zur Aufnahme des chirurgischen Bohrers (4) ausgebildet ist;
wobei das Tiefenkontrollelement (5) und das Instrumentenkörperelement (2) über jeweilige Schnittstellenabschnitte (8, 9) eines drehbaren Zwischenelements (30) miteinander verbunden sind, sodass der zweite Führungskanal (7) proximal zum ersten Führungskanal (3) angeordnet ist, wobei die chirurgische Bohrführung ferner umfasst
- einen Trokar-Einsatz (13), der zur Aufnahme in dem ersten und dem zweiten Führungskanal (3, 7) ausgebildet ist und lösbar mit einem proximalen Abschnitt des Zwischenelements (30) verbunden ist, **dadurch gekennzeichnet, dass**
der Trokar-Einsatz (13) über eine Rastverbindung mit einem proximalen Abschnitt des Zwischenelements (30) verbunden ist, die so ausgelegt ist, dass sie bei einer Trennkraft in proximaler-distaler Richtung löst, die geringer ist als eine Trennkraft, bei der eine Rastverbindung zwischen dem Zwischenelement (30) und dem Instrumentenkörperelement (2) löst.

2. Die chirurgische Bohrführung gemäß Anspruch 1, wobei der Schnittstellenabschnitt (9) des Instrumentenkörperelements (2) so ausgebildet ist, dass er mit dem drehbaren Zwischenelement (30) über ein Drehgelenk in Eingriff steht, wodurch sich das drehbare Zwischenelement (30) relativ zum Instrumentenkörperelement (2) und um eine Drehachse drehen kann, die sich in einer Proximal-Distal-Richtung (D) der Bohrführung (1) erstreckt, und wobei das Drehgelenk die Rastverbindung zwischen dem drehbaren Zwischenelement (30) und dem Instrumentenkörperelement (2) umfasst.

3. Chirurgische Bohrführung nach einem der Ansprüche 1 und 2, wobei
- das drehbare Zwischenelement (30) die Form einer im Wesentlichen zylindrischen Hülse mit einem Innengewinde aufweist;
- die äußere Umfangsfläche des drehbaren Zwischenelements (30) so ausgebildet ist, dass sie eine Greiffläche bildet;
- das drehbare Zwischenelement (30) mindestens ein Sichtfenster aufweist, in dem das Tiefenkontrollelement (5), insbesondere dessen Längenskala, sichtbar ist; und/oder
- der proximale Endabschnitt des drehbaren Zwischenelements (30) einen größeren Innendurchmesser aufweist als der übrige Teil des drehbaren Zwischenelements (30), insbesondere dessen Innengewinde, wodurch der proximale Abschnitt des Tiefenkontrollelements (5) in den proximalen Abschnitt des drehbaren Zwischenelements (30) eintreten kann.

4. Chirurgische Bohrführung nach einem der Ansprüche 1 bis 3, ferner umfassend ein Tracking-Referenzelement (15), wobei das Tracking-Referenzelement (15) und das Instrumentenkörperelement (2) über entsprechende Referenzschnittstellenabschnitte (16, 17), die in mindestens einer Formschlussverbindung sowie in mindestens einer Kraftschlussverbindung miteinander verbunden sind, lösbar miteinander verbunden sind.

5. Chirurgische Bohrführung nach Anspruch 4, wobei mindestens eine Formschlussverbindung eine Position und/oder Ausrichtung definiert, insbesondere genau eine Position und genau eine Ausrichtung, in der das Tracking-Referenzelement (15) und das Instrumentenkörperelement (2) miteinander verbunden sind, und/oder durch entsprechend geformte Oberflächen bereitgestellt wird, an denen die Referenzschnittstellenabschnitte (16, 17) miteinander in Kontakt stehen.

6. Chirurgische Bohrführung nach einem der Ansprüche 4 und 5, wobei die mindestens eine Kraftschlussverbindung umfasst
- eine Schraubverbindung, insbesondere mit einem vom Instrumentenkörperelement (2) bereitgestellten Innengewinde (18), die das Tracking-Referenzelement (15) in Bezug auf das Instrumentenkörperelement (2) in Position hält, und/oder
- an den jeweiligen Referenzschnittstellen (16, 17) angeordnete Magnetelemente (19), die das Tracking-Referenzelement (15) in Bezug auf das Instrumentenkörperelement (2) in Position halten.

7. Chirurgische Bohrführung nach einem der Ansprüche 1 bis 6, ferner umfassend ein Griffelement (20), wobei das Griffelement (20) und das Instrumentenkörperelement (2) über entsprechende Griff-Schnittstellenabschnitte (21, 22) lösbar miteinander verbunden sind, die so ausgebildet sind, dass sie eine Vielzahl von Relativpositionen ermöglichen, insbesondere um eine durch die Griff-Schnittstellenabschnitte (21, 22) definiert ist, wobei das Griffelement (20) mit dem Instrumentenkörperelement (2) verbunden ist.

8. Chirurgische Bohrführung nach Anspruch 7, wobei die Griff-Schnittstellenabschnitte (21, 22)
- um das Tiefenkontrollelement (5) und den darin definierten zweiten Führungskanal (7) herum angeordnet sind, wobei insbesondere der proximale Abschnitt (14) des Instrumentenkörperelements (2) radial zwischen dem Tiefenkontrollelement (5) und den Griff-Anschlussabschnitten (21, 22) angeordnet ist;
- entsprechend geformte Flächen aufweisen, an denen die Griffanschlussabschnitte (21, 22) miteinander in Kontakt stehen, wobei die entsprechend geformten Flächen innerhalb einer Aussparung (23) angeordnet sind, die entweder im Instrumentenkörperelement (2) oder im Griffelement (20) ausgebildet ist; und/oder
- in einer Formschlussverbindung miteinander verbunden sind, die eine vordefinierte Anzahl relativer Positionen zulässt.

9. Chirurgische Bohrführung gemäß einem der Ansprüche 7 und 8, wobei die Griffverbindungsabschnitte (21, 22) durch einen Mechanismus zusammengehalten werden, der einen Knopf (24), der über eine Bajonettkupplung (25) mit dem Instrumentenkörperelement (2) in Eingriff steht, und ein Federelement (26) umfasst, das den Knopf (24) mit dem Griffelement (20) verbindet, wobei der Mechanismus so ausgelegt ist, dass er das Federelement (26) vorspannt, wenn der Knopf (24) in das Instrumentenkörperelement (2) eingreift, wodurch die Griff-Anschlussabschnitte (21, 22) zusammengedrückt werden, insbesondere wobei der Knopf (24) an einer radialen Position des offenen Querschnitts (12) des zweiten Führungskanals (7) eine Abschirmung (27) aufweist.

10. Chirurgische Bohrführung gemäß einem der Ansprüche 1 bis 9, wobei der erste Führungskanal (3) durch ein Instrumentenspitzenelement (28) gebildet wird, das lösbar mit dem Instrumentenkörperelement (2) verbunden ist, insbesondere wobei ein federbelasteter Mechanismus (29) das Instrumentenspitzenelement (28) über eine Formschlussverbindung in Bezug auf das Instrumentenkörperelement (2) in Position hält.

11. Bohrführungssystem, umfassend
- eine chirurgische Bohrführung (1) gemäß einem der Ansprüche 1 bis 10;
- zwei oder mehr Tiefenkontrollelemente (5), die zur Verbindung mit dem Instrumentenkörperelement (2) ausgelegt sind, und/oder zwei oder mehr Instrumentenspitzenelemente (28), die zur Verbindung mit dem Instrumentenkörperelement (2) ausgelegt sind;
- zwei oder mehr chirurgische Bohrer (4);
wobei die ersten Führungskanäle (3), die jeweils von den mindestens zwei Tiefenkontrollelementen (5) bereitgestellt werden, und/oder die zweiten Führungskanäle (7), die jeweils von den mindestens zwei Instrumentenspitzenelementen (28) bereitgestellt werden, Innendurchmesser aufweisen, die dem Außendurchmesser eines proximalen Schaftabschnitts und/oder dem Außendurchmesser eines distalen Schneidabschnitts des mindestens einen Bohrers (4) entsprechen, sodass nur bestimmte Arten von chirurgischen Bohrern (4), die insbesondere eine bestimmte Länge aufweisen, in dem ersten Führungskanal (3) und/oder dem zweiten Führungskanal (7) aufgenommen werden können.

## Revendications

1. Guide de perçage chirurgical comprenant :
- un élément de corps d'instrument (2) présentant un premier canal de guidage (3) conçu pour recevoir un foret chirurgical (4), et
- un élément de réglage de profondeur (5) présentant une surface de butée de foret proximale (6) et un deuxième canal de guidage (7) conçu pour recevoir le foret chirurgical (4) ; l'élément de réglage de profondeur (5) et l'élément de corps d'instrument (2) étant reliés l'un à l'autre par l'intermédiaire de portions d'interface (8, 9) respectives d'un élément intermédiaire rotatif (30) permettant au deuxième canal de guidage (7) d'être disposé du côté proximal du premier canal de guidage (3), le guide de perçage chirurgical comprenant en outre
- un insert de trocart (13) conçu pour être reçu dans le premier et le deuxième canal de guidage (3, 7) et qui se raccorde de manière amovible à une portion proximale de l'élément intermédiaire (30) ; **caractérisé en ce que**
l'insert de trocart (13) se raccorde à une portion proximale de l'élément intermédiaire (30) par l'intermédiaire d'un raccord à encliquetage apte à se déverrouiller sous l'effet d'une force de séparation à orientation proximale-distale, laquelle est inférieure à une force de séparation ayant pour effet de déverrouiller un raccord à encliquetage entre l'élément intermédiaire (30) et l'élément de corps d'instrument (2).

2. Guide de perçage chirurgical selon la revendication 1, dans lequel la portion d'interface (9) de l'élément de corps d'instrument (2) est conçue pour s'accoupler avec l'élément intermédiaire rotatif (30) par le biais d'un emboîtement rotatif permettant à l'élément intermédiaire rotatif (30) de tourner par rapport à l'élément de corps d'instrument (2) et sur un axe de rotation s'étendant dans une direction proximale-distale (D) du guide de perçage (1), et l'emboîtement rotatif comprend le raccord à encliquetage entre l'élément intermédiaire rotatif (30) et l'élément de corps d'instrument (2).

3. Guide de perçage chirurgical selon l'une quelconque des revendications 1 et 2, dans lequel
- l'élément intermédiaire rotatif (30) présente la forme d'un manchon sensiblement cylindrique présentant un filetage intérieur,
- la surface circonférentielle extérieure de l'élément intermédiaire rotatif (30) est conçue pour fournir une surface de préhension,
- l'élément intermédiaire rotatif (30) comprend au moins une fenêtre d'inspection dans laquelle l'élément de réglage de profondeur (5), notamment une échelle de longueur de celui-ci, est visible, et/ou
- la portion d'extrémité proximale de l'élément intermédiaire rotatif (30) présente un diamètre intérieur plus grand que le reste de l'élément intermédiaire rotatif (30), notamment le filetage intérieur de celui-ci, ce qui permet à la portion proximale de l'élément de réglage de profondeur (5) de pénétrer dans la portion proximale de l'élément intermédiaire rotatif (30).

4. Guide de perçage chirurgical selon l'une quelconque des revendications 1 à 3, comprenant en outre un élément de référence de suivi (15), l'élément de référence de suivi (15) et l'élément de corps d'instrument (2) se raccordant de manière déverrouillable l'un à l'autre par l'intermédiaire de portions d'interface de référence (16, 17) correspondantes s'assemblant au moins par un raccordement par complémentarité de forme et au moins par un raccordement par adhérence.

5. Guide de perçage chirurgical selon la revendication 4, dans lequel au moins un raccordement par complémentarité de forme définit un emplacement et/ou une orientation, notamment exactement un emplacement et exactement une orientation permettant à l'élément de référence de suivi (15) et à l'élément de corps d'instrument (2) de se raccorder l'un à l'autre et/ou est fourni par des surfaces de formes correspondantes au niveau desquelles les portions d'interface de référence (16, 17) entrent en contact entre elles.

6. Guide de perçage chirurgical selon l'une quelconque des revendications 4 et 5, dans lequel l'au moins un raccordement par adhérence comprend
- un raccordement par vissage, notamment au moyen d'un filetage intérieur (18) prévu sur l'élément de corps d'instrument (2), maintenant l'élément de référence de suivi (15) en place par rapport à l'élément de corps d'instrument (2) et/ou
- des éléments magnétiques (19) disposés au niveau des portions d'interface de référence (16, 17) respectives, maintenant l'élément de référence de suivi (15) en place par rapport à l'élément de corps d'instrument (2).

7. Guide de perçage chirurgical selon l'une quelconque des revendications 1 à 6, comprenant en outre un élément de poignée (20), ledit élément de poignée (20) et l'élément de corps d'instrument (2) se raccordant de manière déverrouillable l'un à l'autre par l'intermédiaire de portions d'interface de poignée (21, 22) correspondantes qui sont conçues pour permettre une pluralité de positions relatives, notamment sur un axe de rotation (R) défini par les portions d'interface de poignée (21, 22), selon lesquelles l'élément de poignée (20) est raccordé à l'élément de corps d'instrument (2).

8. Guide de perçage chirurgical selon la revendication 7, dans lequel les portions d'interface de poignée (21, 22)
- sont disposées autour de l'élément de réglage de profondeur (5) et du deuxième canal de guidage (7) défini dans celui-ci, la portion proximale (14) de l'élément de corps d'instrument (2) étant notamment disposée radialement entre l'élément de réglage de profondeur (5) et les portions d'interface de poignée (21, 22),
- comprennent des surfaces de forme correspondante au niveau desquelles les portions d'interface de poignée (21, 22) entrent en contact entre elles, les surfaces de forme correspondante étant disposées à l'intérieur d'un évidement (23) formé soit dans l'élément de corps d'instrument (2), soit dans l'élément de poignée (20), et/ou
- s'assemblent par un raccordement par complémentarité de forme permettant un nombre prédéfini de positions relatives.

9. Guide de perçage chirurgical selon l'une quelconque des revendications 7 et 8, dans lequel les portions d'interface de poignée (21, 22) sont maintenues ensemble par un mécanisme comprenant une molette (24) s'accouplant avec l'élément de corps d'instrument (2) par l'intermédiaire d'un raccord à baïonnette (25), et un élément à ressort (26) raccordant la molette (24) et l'élément de poignée (20), le mécanisme étant conçu pour précontraindre l'élément à ressort (26) tandis que la molette (24) s'accouple avec l'élément de corps d'instrument (2), forçant ainsi les portions d'interface de poignée (21, 22) à se rapprocher l'une de l'autre, la molette (24) fournissant une protection (27) en une position radiale de la section transversale ouverte (12) du deuxième canal de guidage (7).

10. Guide de perçage chirurgical selon l'une quelconque des revendications 1 à 9, dans lequel le premier canal de guidage (3) est fourni par un élément d'embout d'instrument (28) qui se raccorde de manière déverrouillable à l'élément de corps d'instrument (2), un mécanisme à ressort comprimé (29) maintenant notamment l'élément d'embout d'instrument (28) en place par rapport à l'élément de corps d'instrument (2) par l'intermédiaire d'un raccordement par complémentarité de forme.

11. Système de guidage de forage comprenant
- un guide de perçage chirurgical (1) selon l'une quelconque des revendications 1 à 10,
- au moins deux éléments de réglage de profondeur (5) aptes à se raccorder à l'élément de corps d'instrument (2) et/ou au moins deux éléments d'embout d'instrument (28) aptes à se raccorder à l'élément de corps d'instrument (2),
- au moins deux forets chirurgicaux (4) ;
les premiers canaux de guidage (3) respectivement fournis par les au moins deux éléments de réglage de profondeur (5) et/ou les deuxièmes canaux de guidage (7) respectivement fournis par les au moins deux éléments d'embout d'instrument (28) présentant des diamètres intérieurs qui correspondent au diamètre extérieur d'une portion de tige proximale et/ou au diamètre extérieur d'une portion de coupe distale de l'au moins un foret (4), permettant que seuls des types spécifiques de forets chirurgicaux (4), qui présentent notamment une longueur spécifique, puissent être reçus dans le premier canal de guidage (3) et/ou le deuxième canal de guidage (7).
